# EUROPEAN PATENT APPLICATION

(11) **EP 4 840 391 A1**
(43) Date of publication of application: **18.11.2026**
(21) Application number: 26177991.2
(22) Date of filing: 11.05.2026
(51) Int. Cl.: A61B 5/00, A61B 5/0205, G16H 40/63, G16H 50/30

(54) **MEDICAL DEVICE WITH CONFIGURABLE ALARM PAUSE**

(30) Priority: 13.05.2025 US 202563804773 P
(71) Applicant: Welch Allyn, INC., Skaneateles Falls, NY 13153-0220 (US)
(72) Inventor: MYERS, Thomas A., Skaneateles Falls, 13153-0220 (US); COLES, Kathryn M., Skaneateles Falls, 13153-0220 (US); DRUM PAQUETTE, Hannah, Skaneateles Falls, 13153-0220 (US); ENDRES, Danielle R., Skaneateles Falls, 13153-0220 (US); HAYWARD, Lauren N., Skaneateles Falls, 13153-0220 (US); MCSWEENEY, WonKyung, Skaneateles Falls, 13153-0220 (US); QUILTY-KOVAL, Rebecca, Skaneateles Falls, 13153-0220 (US)
(74) Representative: Reddie & Grose LLP

(57) **Abstract**

A medical device for monitoring one or more physiological variables controls a speaker to emit an audible alarm when an alarm condition is detected. In response to receiving a first input, the medical device displays a menu that includes at least a first alarm pause having a first duration and a second alarm pause having a second duration different from the first duration. In response to receiving a second input, the medical device controls the speaker to pause the audible alarm for the first duration or the second duration.

## Description

Nuisance alarms in medical devices are false or non-urgent alerts that can cause unnecessary disruptions in healthcare environments. These alarms, while designed to notify healthcare providers of potential issues, often arise from settings that are too sensitive, improper configurations, or routine fluctuations that do not require immediate attention. The constant barrage of nuisance alarms can lead to alarm fatigue, where healthcare professionals may begin to overlook or ignore alarms, potentially jeopardizing patient safety. Addressing nuisance alarms is crucial in improving the efficiency and effectiveness of medical device systems, ensuring that only critical alerts grab the attention of healthcare professionals.

In general terms, the present disclosure relates to medical device alarm management. In one possible configuration, a medical device is configurable to pause an audible alarm for one or more customized durations. Various aspects are described in this disclosure, which include, but are not limited to, the following aspects.

One aspect relates to a medical device for monitoring one or more physiological variables, the medical device comprising: a display configured to display measurements of one or more physiological variables; a speaker configured to emit an audible alarm when the measurements of the one or more physiological variables are higher than an upper alarm limit or when the measurements of the one or more physiological variables are lower than a lower alarm limit; at least one processing device communicatively coupled to the display, the speaker, and at least one sensor that captures the measurements of the one or more physiological variables; and a memory device storing instructions which, when executed by the at least one processing device, cause the at least one processing device to: control the speaker to emit the audible alarm when the measurements of the one or more physiological variables are higher than the upper alarm limit or when the measurements of the one or more physiological variables are lower than the lower alarm limit; in response to receiving a first input, display a menu that includes at least a first alarm pause having a first duration and a second alarm pause having a second duration that is different from the first duration; and in response to receiving a second input, control the speaker to pause the audible alarm for the first duration or the second duration.

Another aspect relates to a medical device for monitoring one or more physiological variables, the medical device comprising: a display configured to display measurements of the one or more physiological variables; a user presence sensor; a speaker configured to emit an audible alarm when measurements of the one or more physiological variables are higher than an upper alarm limit or when the measurements of the one or more physiological variables are lower than a lower alarm limit; at least one processing device communicatively coupled to the user presence sensor, the speaker, and at least one sensor that captures the measurements of the one or more physiological variables; and a memory device storing instructions which, when executed by the at least one processing device, cause the at least one processing device to: detect a presence of an authorized user within a threshold distance of the medical device based on detection of a signal by the user presence sensor; control the speaker to pause the audible alarm for an extended duration when the authorized user remains within the threshold distance of the medical device; and resume a default alarm pause when the authorized user moves outside of the threshold distance of the medical device.

Another aspect relates to a method of monitoring one or more physiological variables, the method comprising: controlling a speaker to emit an audible alarm when measurements of the one or more physiological variables are higher than an upper alarm limit or when the measurements of the one or more physiological variables are lower than a lower alarm limit; in response to receiving a first input, displaying a menu that includes at least a first alarm pause having a first duration and a second alarm pause having a second duration that is different from the first duration; and in response to receiving a second input, controlling the speaker to pause the audible alarm for the first duration or the second duration.

The invention will now be further described by way of example with reference to the accompanying drawings, in which:
FIG. 1 is a front isometric view of a medical device.
FIG. 2 is a rear isometric view of the medical device of FIG. 1.
FIG. 3 is a front view of the medical device of FIG. 1.
FIG. 4 is a bottom view of the medical device of FIG. 1.
FIG. 5 schematically illustrates an example of the medical device of FIG. 1.
FIG. 6 schematically illustrates an example of a method of managing audible alarms on the medical device of FIG. 1.
FIG. 7 illustrates an example of a home screen user interface that can be displayed on a display of the medical device of FIG. 1.
FIG. 8 illustrates an example of a home screen user interface that can be displayed on the display of the medical device of FIG. 1 in accordance with the method of FIG. 6.
FIG. 9 illustrates another example of a home screen user interface that can be displayed on the display of the medical device of FIG. 1 as a result of a selection of an alarm pause having an extended duration in an alarm pause menu displayed in FIG. 8.
FIG. 10 illustrates an example of a configuration user interface that can be displayed on the display to configure an alarm pause tool for execution on the medical device of FIG. 1.
FIG. 11 schematically illustrates another example of a method of managing audible alarms on the medical device of FIG. 1.

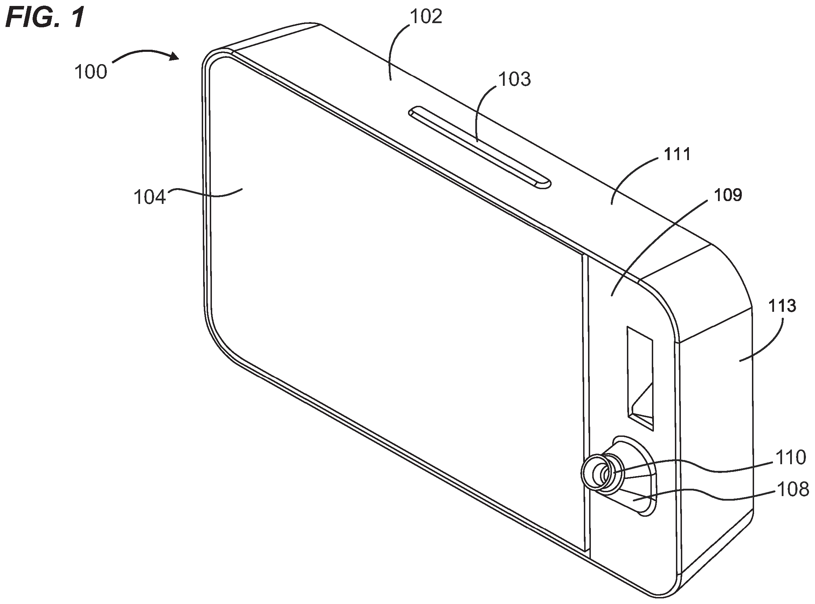

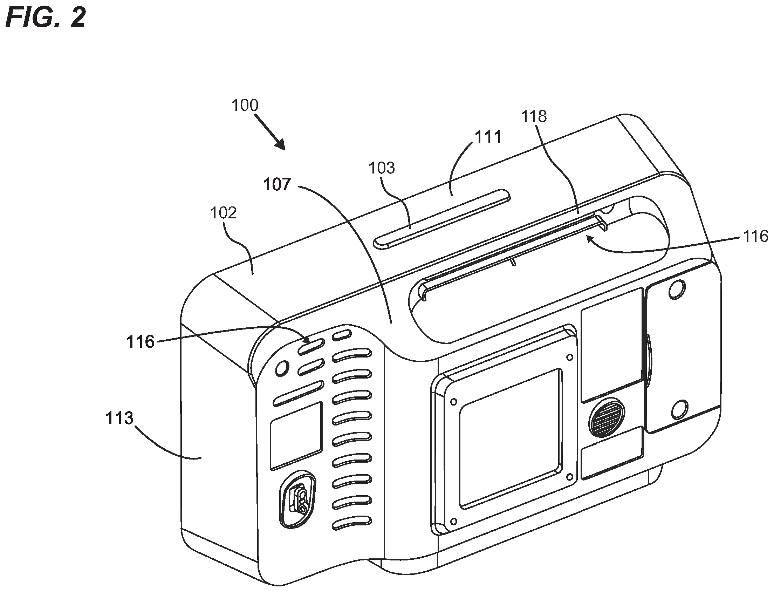

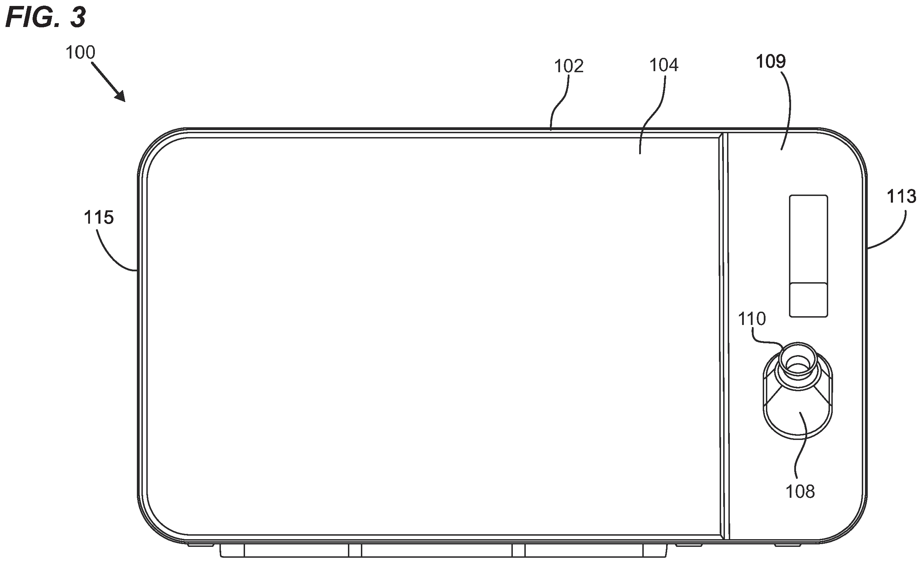

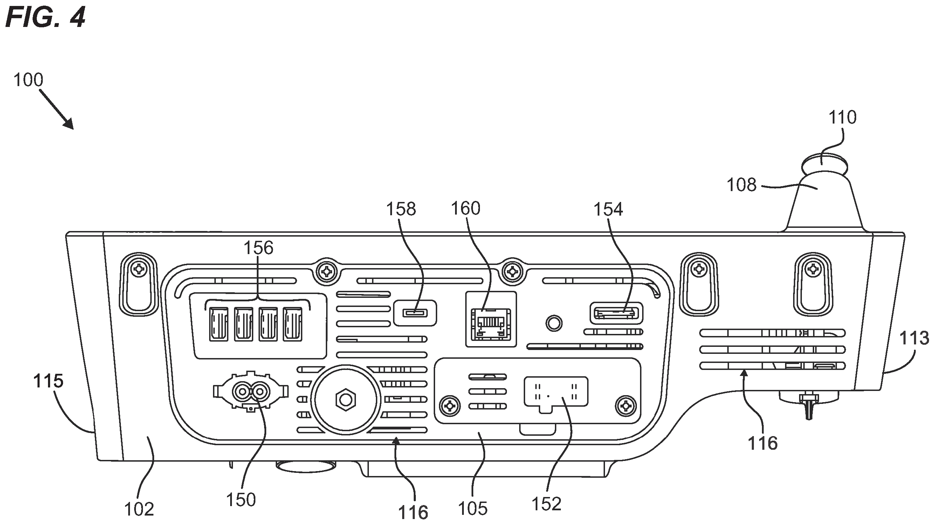

FIG. 1 is a front isometric view of a medical device 100 for monitoring one or more physiological variables of a patient. FIG. 2 is a rear isometric view of the medical device 100. FIG. 3 is a front view of the medical device 100. FIG. 4 is a bottom view of the medical device 100. As shown in FIGS. 1-4, the medical device 100 includes a housing 102 having a bottom surface 105, a rear surface 107, a front surface 109, a top surface 111, and lateral sides 113, 115 which give the medical device 100 a substantially rectangular prism shape. The industrial design shown in FIGS. 1-4 is illustrative and may vary in different examples of the medical device 100.

In some examples, the medical device 100 is portable. In such examples, the medical device 100 can include a handle 118 (see FIG. 2) that allows the housing 102 to be gripped with one hand for physically moving the medical device 100 from one location to another.

A display 104 is mounted on a front surface 109 of the housing 102. In some examples, the display 104 is a touchscreen that operates to both display data captured by one or more sensors communicatively coupled to the medical device 100 and to receive tactile inputs from a user such as to adjust one or more alarm parameters and/or to silence an alarm. The housing 102 can also support physical push buttons to receive tactile inputs from the user.

The medical device 100 is designed for use in a clinical setting. For example, the medical device 100 can be used to continuously monitor one or more physiological variables of a patient admitted to a medical facility such as a hospital, a medical clinic, and the like. Additionally, the medical device 100 can operate as a spot monitor for episodic monitoring of one or more physiological variables of a plurality of patients in the medical facility.

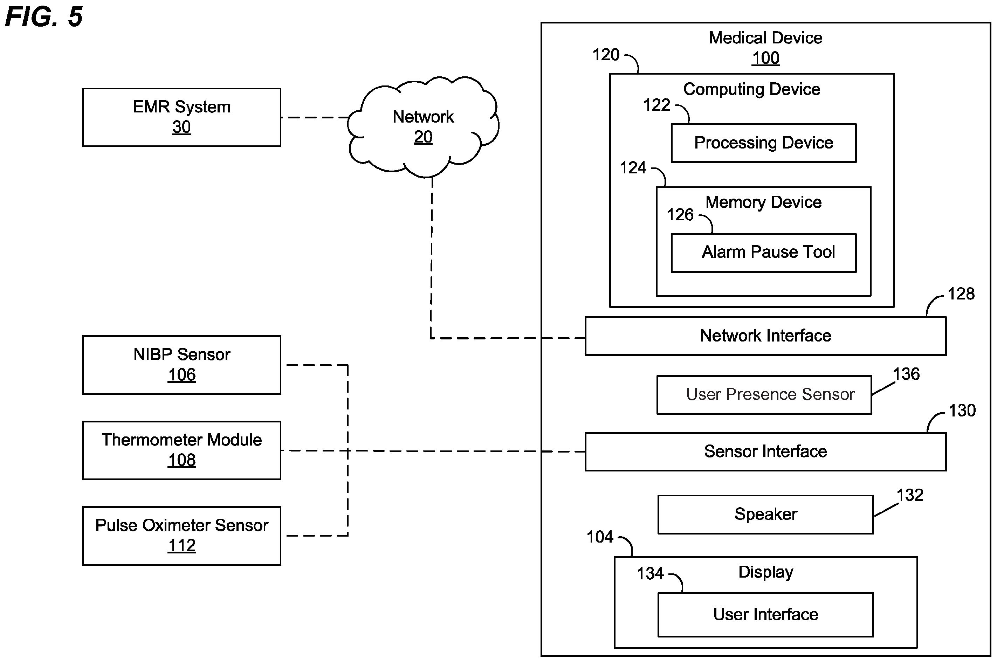

The medical device 100 is configured for connection to one or more sensors (see FIG. 5) that can be used to measure one or more physiological variables including, without limitation, non-invasive systolic and diastolic blood pressure, blood oxygen saturation (Sp02), respiration rate, pulse rate, and body temperature. For example, as shown in FIGS. 1, 3, and 4, the sensors can include a thermometer module 108 that can be used to measure body temperature.

In the example shown in FIGS. 1, 3, and 4, the thermometer module 108 is integrated with the medical device 100. The thermometer module 108 includes a port 110 for housing a handheld probe that can be orally inserted into the mouth of a patient to take a temperature reading. Alternatively, the housing 102 of the medical device 100 can be configured to house a handheld probe that can be inserted into an ear of the patient to take a temperature reading.

As shown in FIG. 5, which will be described in more detail further below, the sensors can include a non-invasive blood pressure (NIBP) sensor 106 that includes a cuff that can be inflated around the arm of a patient to non-invasively measure systolic and diastolic blood pressure. The sensors can further include a pulse oximeter sensor 112 that can attach to a finger of the patient to measure vital signs including one or more of blood oxygen saturation (SpO2), pulse rate, and respiration rate. It is contemplated that the medical device 100 can interface with additional types of sensors for measuring additional types of physiological variables.

The sensors can include cables that terminate into connectors that plug into various ports included on the housing 102 of the medical device 100 for transmission of data to the medical device 100. As shown in the example of FIG. 4, the housing 102 includes a first port 150 for connecting the NIBP sensor 106 and a second port 152 for connecting the pulse oximeter sensor 112. The medical device 100 can include a third port 154 for connecting the medical device 100 to a printer. Also, the medical device 100 can include one or more universal serial bus (USB) ports 156, a USB-C port 158, and a registered jack (RJ) interface 160. In some examples, at least some of the sensors wirelessly transmit data to the medical device 100 via a wireless connection established through Wi-Fi, Bluetooth, or another wireless protocol.

In some examples, a scanner can be connected to the medical device 100 such as by plugging into a USB port 156. The scanner can be used to scan machine-readable data such as barcodes, quick-response (QR) codes, and the like that identify a person such as a patient who is being monitored by the medical device 100 or a user of the medical device 100 (e.g., a nurse, a doctor, a caregiver, and the like). The machine-readable data can further identify an object such as medical equipment or a medication being administered to the patient.

The medical device 100 receives data from the sensors for processing. The medical device 100 generates and displays numerical values and/or waveforms based on the data received from the sensors for monitoring the one or more physiological variables of the patient. The medical device 100 displays the numerical values and/or waveforms of the measurements of one or more physiological variables on the display 104. Also, the medical device 100 generates alarms when the data received from the sensors indicates an alarm condition is detected.

The alarms generated by the medical device 100 can include a visual alarm displayed on the display 104 and an audible alarm emitted by a speaker 132 of the medical device 100. An additional visual alarm can be displayed on an illumination unit 103, which is positioned on the top surface 111 of the housing 102 of the medical device 100. The illumination unit 103 allows for distant viewing of critical alarms. In some examples, a visual alarm is displayed on the display 104 and a second visual alarm is generated on the illumination unit 103 to indicate a critical status of a detected alarm condition. The combination of the visual alarm on the illumination unit 103 and the visual alarm displayed on the display 104 allows critical visual alarms to be viewed across a 360 degree field of view around the medical device 100.

The illumination unit 103 can include one or more light-emitting diodes (LEDs) that emit light that is visible through a translucent cover. As an illustrative example, the illumination unit 103 can emit light having a color (e.g., yellow, red, or other colors) based on a severity of the alarm. Additionally, or alternatively, the illumination unit 103 can emit light flashes based on a predetermined pattern associated with the severity of the alarm (e.g., an increased blinking rate to indicate severe conditions and a decreased blinking rate to indicate less severe conditions).

As further shown in the example of the medical device 100 illustrated in FIGS. 1-4, the housing 102 further includes a plurality of vents 116 for dissipating hot air away from internal components of the medical device. The vents 116 are positioned at different areas of the housing 102 such as on the bottom surface 105 (FIG. 4) and the rear surface 107 (FIG. 2).

FIG. 5 schematically illustrates an example of the medical device 100. In this illustrative example, the medical device 100 includes a computing device 120 having at least one processing device 122 and at least one memory device 124 that stores software instructions that, when executed by the at least one processing device 122, cause the at least one processing device 122 to perform the various aspects, functions, and operations described herein. The at least one processing device 122 is communicatively coupled to at least the display 104, to the speaker 132, and to at least one sensor that captures measurements of a physiological variable.

The at least one processing device 122 is an example of a processing unit such as a central processing unit (CPU). The at least one processing device 122 can include one or more CPUs. In some examples, the at least one processing device 122 includes one or more digital signal processors, field-programmable gate arrays, or other electronic circuits.

The at least one memory device 124 is an example of a computer-readable data storage device that operates to store data and instructions for execution by the at least one processing device 122. The at least one memory device 124 includes computer-readable media, which includes any media that can be accessed by the at least one processing device 122.

The at least one memory device 124 stores an alarm pause tool 126 that when executed by the at least one processing device 122 allows a user of the medical device 100 to choose an alarm audio pause time longer than a default alarm audio pause. The alarm pause tool 126 displays a plurality of available alarm audio pause time options on the display 104 that are configurable and that are limited to a maximum number of minutes defined by an administrator. The alarm pause tool 126 can be used by a clinician to minimize distractions during assessment and treatment of a patient such as at the bedside. Extended pause can be canceled early at any time by the clinician. The alarm pause tool 126 will be described in more detail further below.

The computer-readable media can include computer-readable storage media and computer-readable communication media. The computer-readable storage media includes volatile and nonvolatile, removable and non-removable media implemented in any device that stores information such as computer-readable instructions, data structures, program modules, or other data. The computer-readable storage media can include random access memory, read only memory, electrically erasable programmable read only memory, flash memory, and other memory technology, including any medium that can be used to store information for access by the at least one processing device 122. The computer-readable storage media is non-transitory.

The computer-readable communication media embodies computer-readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. The computer-readable communication media can include wired media such as a wired network or direct-wired connection, and wireless media such as acoustic, radio frequency, infrared, and other wireless media. Combinations of any of the above are within the scope of computer-readable media.

As further shown in FIG. 5, the medical device 100 includes a network interface 128 that facilitates connection to a network 20 that can include any type of wired or wireless connections or any combinations thereof. The network interface 128 can include wired interfaces and/or wireless interfaces. For example, the network interface 128 can wirelessly connect the medical device 100 to the network 20 such as through Wi-Fi. Alternatively, or additionally, the network interface 128 can connect the medical device 100 to the network 20 using wired connections such as through Ethernet or Universal Serial Bus (USB) cables.

The network interface 128 enables the medical device 100 to communicate via the network 20 with an electronic medical record (EMR) system 30. The EMR system 30 maintains the medical history of a plurality of patients by consolidating health information for each patient into a single document called an electronic medical record (EMR). Each EMR can include patient demographic information, medical diagnoses, treatment plans, medications, test results, physiological variable measurements, and immunization history. The EMR system 30 enables sharing of data among healthcare professionals to improve coordination of care, reduce errors, and enhance patient outcomes. The EMR system 30 also supports clinical decision-making by providing real-time access to comprehensive patient health information. The EMR system 30 can include secure data storage and integration with other healthcare technologies, which streamline administrative tasks and improve overall healthcare efficiency.

The medical device 100 further includes a sensor interface 130 that operates to communicate with the various sensors that can be communicatively coupled to the medical device 100 such as the NIBP sensor 106, the thermometer module 108, and the pulse oximeter sensor 112. The sensor interface 130 can include both wired interfaces and wireless interfaces. Examples of wireless interfaces can include Wi-Fi, Bluetooth, and other wireless connections. Examples of wired interfaces can include the ports of the medical device 100 (see FIG. 4) that allow connectorized ends of USB or Ethernet cables to plug into the sensor interface 130.

The speaker 132 is an integral component of the medical device 100 for providing clear auditory feedback for users and healthcare professionals. The speaker 132 is used to deliver audible alarms, alerts, notifications, and/or instructions. For example, the speaker 132 emits an audible alarm when the measurements of a physiological variable are higher than an upper alarm limit or when the measurements of the physiological variable are lower than a lower alarm limit. The speaker 132 ensures that vital information such as detected alarm conditions is communicated effectively. The speaker 132 enhances patient safety by making sure critical data is heard, even in busy or noisy environments which are typical in medical settings. The speaker 132 is communicatively coupled to the at least one processing device 122.

As shown in FIG. 5, the medical device 100 includes the display 104 which operates to display user interfaces 134. The display 104 can include a touch sensitive screen (e.g., a touchscreen) that receives tactile inputs from the touch of a finger or stylus on its surface. In such examples, the display 104 can include a grid of capacitive or resistive sensors to register locations of touch. In some examples, the display 104 includes a capacitive touchscreen that is coated with a conductive material. When a finger or stylus touches the capacitive touchscreen, a local electrostatic field produced by the conductive material of the display 104 changes, allowing the medical device 100 to determine a point of contact. Alternatively, the display 104 can include a resistive touchscreen that registers pressure applied to the screen by detecting change in resistance when layers of the screen come into contact. The tactile inputs, whether capacitive or resistive, are processed by the computing device 120 to identify actions such as selecting or dragging visual objects that are displayed on the user interfaces 134.

The medical device 100 can include a user presence sensor 136 that detects wireless signals from external devices that are in proximity to the medical device 100. The user presence sensor 136 allows the medical device 100 to detect the presence of external devices such as wearable badges that are associated with authorized users of the medical device 100. For example, the user presence sensor 136 can be used to detect the presence of an authorized user within a threshold distance of the medical device 100 based on the wireless signals received from the external device associated with the authorized user. Further, the user presence sensor 136 allows the medical device 100 to wirelessly communicate with the external device such as to receive commands or instructions from the external device, and/or to send data to the external device relevant to the status of a patient being monitored by the medical device 100. The user presence sensor 136 can be configured to detect, without limitation, Wi-Fi, Bluetooth, Zigbee, radio frequency identification (RFID), Z-Wave, LoRaWAN, or other wireless signals.

Alarm fatigue can become problematic, especially when one or more physiological variables are being continuously monitored by the medical device 100. Alarm fatigue can lead to heightened clinician and patient stress which can negatively affect patient care and health outcomes. A clinician can pause an audible alarm on the medical device 100 for a default duration. However, when the default duration for pausing the alarm expires while the clinician is still caring for the patient, the return of the audible alarm can cause a distraction to both the patient and the clinician that affects the quality of healthcare.

The alarm pause tool 126 addresses the foregoing technical problems by enabling selection of an audible alarm pause duration that is longer than the duration of a default audible alarm pause. The alarm pause tool 126 displays a plurality of available durations on the display 104 that are selectable to pause the audible alarm to minimize distractions during treatment of a patient at the bedside. The extended alarm pause can be canceled early at any time by the user.

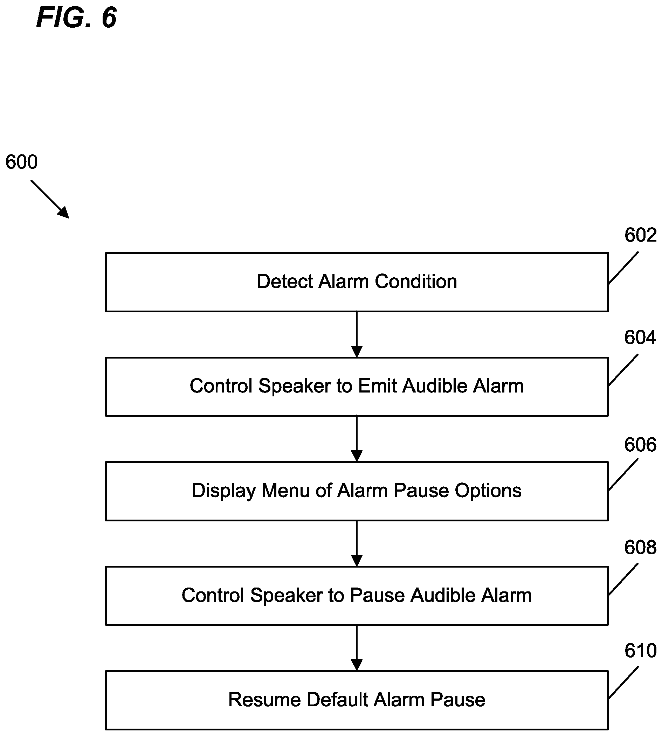

FIG. 6 schematically illustrates an example of a method 600 of managing audible alarms on the medical device 100. The method 600 can be performed by the alarm pause tool 126 executed on the medical device 100. The method 600 can be executed on the medical device 100 to pause audible alarms triggered by any physiological measurement monitored by the medical device 100 including, without limitation, one or more measurements of blood oxygen saturation (SpO2), pulse rate, respiration rate, and non-invasive blood pressure (NIBP).

The method 600 includes an operation 602 of detecting an alarm condition based on data from one or more sensors communicatively coupled to the medical device 100 such as the NIBP sensor 106, the thermometer module 108, and the pulse oximeter sensor 112. Operation 602 can include determining measurement values of the one or more physiological variables based on the data received from the sensors communicatively coupled to the medical device 100, and comparing the measurement values to upper and lower alarm limits. For example, operation 602 can include detecting an alarm condition when the measurement values exceed the upper alarm limit or when the measurement values are less than the lower alarm limit.

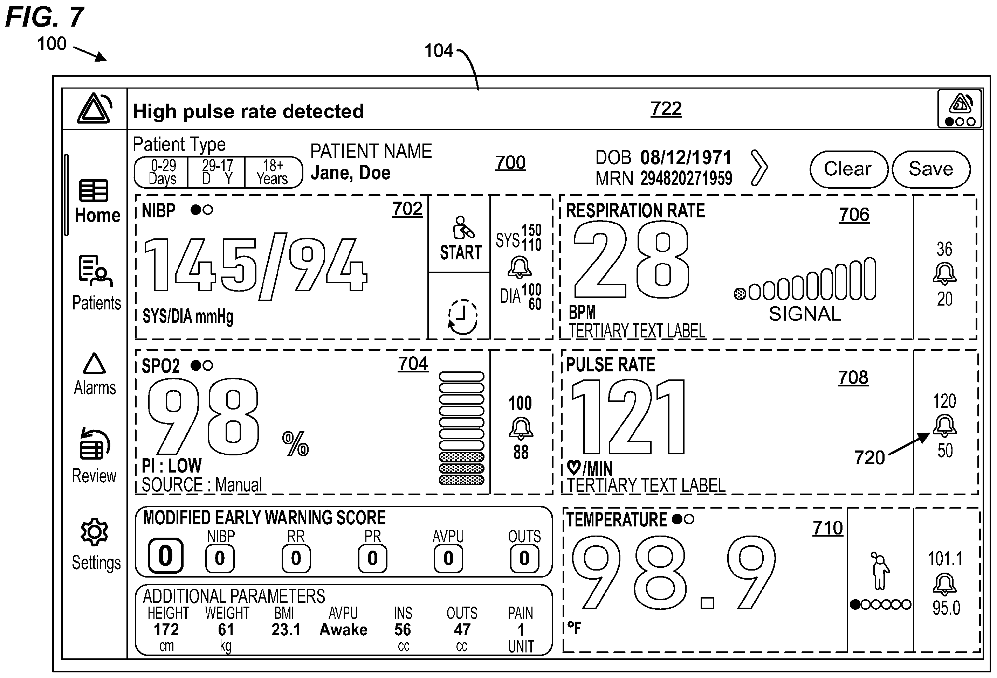

FIG. 7 illustrates an example of a home screen user interface 700 that can be displayed on the display 104 of the medical device 100. In this illustrative example, the home screen user interface 700 includes a non-invasive blood pressure (NIBP) display area 702 that displays systolic and diastolic blood pressure measurements based on the data received from the NIBP sensor 106. The home screen user interface 700 further includes a blood oxygen saturation (SpO2) display area 704 that displays SpO2 measurements, a respiration rate display area 706 that displays respiration rate measurements, and a pulse rate display area 708 that displays pulse rate measurements based on the data received from the pulse oximeter sensor 112. The home screen user interface 700 further includes a temperature display area 710 that displays temperature measurements based on data from the thermometer module 108.

In this illustrative example, an alarm condition is detected based on the pulse rate measurements displayed in the pulse rate display area 708. The pulse rate display area 708 displays a pulse rate measurement of 121 beats per minute (bpm) which exceeds an upper alarm limit of 120 bpm. In this example, a lower alarm limit of 50 bpm is set for the pulse rate.

As shown in FIG. 7, when the alarm condition is detected by the medical device 100, a border of the display area with the detected alarm condition, in this case the pulse rate display area 708, is illuminated (e.g., in the color red) to distinguish the display area from the other display areas of the home screen user interface 700. Also, an alarm icon 720 is illuminated (e.g., in the color red) inside the display area with the alarm condition. Further, an alarm ribbon 722 is displayed across the home screen user interface 700 such as across a top portion of the interface. The alarm ribbon 722 identifies the alarm condition (e.g., "High pulse rate detected").

Referring back to FIG. 6, the method 600 includes an operation 604 of controlling the speaker 132 to emit an audible alarm based on the alarm condition detected in operation 602. For example, when the measurements of a physiological variable are higher than an upper alarm limit or when the measurements of a physiological variable are lower than a lower alarm limit, the speaker 132 is controlled to emit an audible alarm to alert a user of the medical device 100 such as a clinician, nurse, physician, or other type of caregiver about the alarm condition.

In further examples, a score calculated based on one or more physiological variable measurements can trigger the audible alarm when the score exceeds a threshold. In some examples, the score is a composite score that is derived from combining individual scores that are calculated based on various physiological variables and/or parameters. In some examples, the score is an early warning score that assesses a patient's overall condition or risk for deterioration.

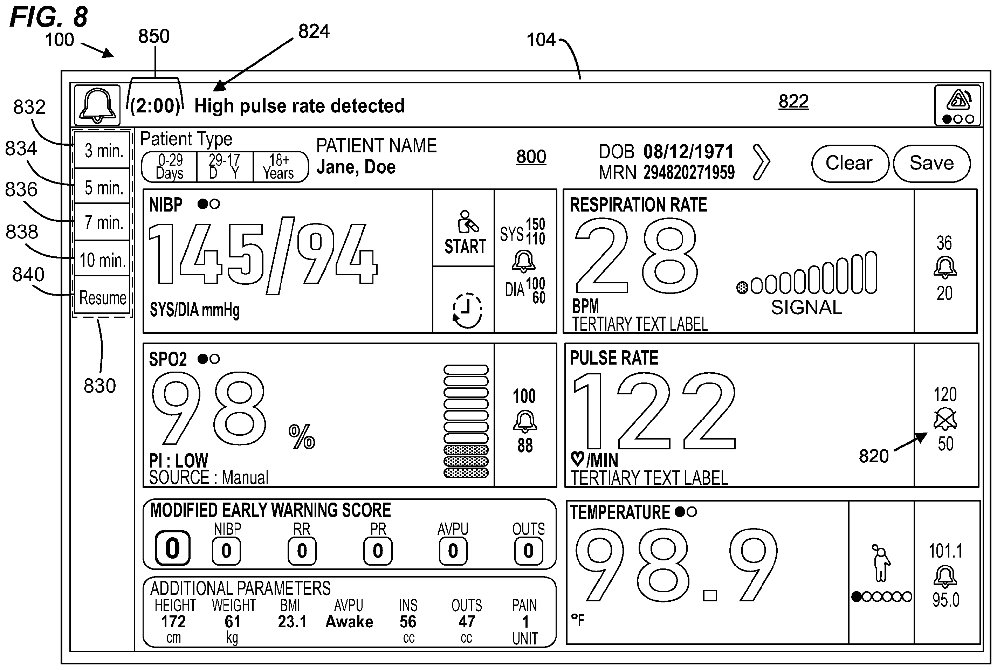

In response to receiving an input, the method 600 includes an operation 606 of displaying a menu that includes at least a first alarm pause having a first duration and a second alarm pause having a second duration that is different from the first duration. The first and second durations are examples of extended audible alarm pauses where the first and second durations are each longer than a default duration (e.g., 2 minutes) for a default alarm pause 824 (see FIG. 8). In some examples, the input is a tactile input that is detected on the display 104.

As an illustrative example, the tactile input can be received on the alarm icon 720 or the alarm ribbon 722 (see FIG. 7), which are selectable by touching the display 104 with a finger or stylus. Upon receipt of the tactile input on the alarm icon 720 or the alarm ribbon 722, the default alarm pause 824 having the default duration (e.g., 2 minutes) is automatically activated on the medical device 100 while the menu is displayed on the display 104 for selecting an extended audible alarm pause having a duration longer than the default duration.

FIG. 8 illustrates an example of a home screen user interface 800 that can be displayed on the display 104 of the medical device 100 in accordance with operation 606 of the method 600. As shown in FIG. 8, an alarm pause menu 830 is displayed as a result of receiving a tactile input on the alarm icon 820 or the alarm ribbon 822 in the home screen user interface 800.

The default alarm pause 824 having the default duration (e.g., two minutes) is applied automatically for silencing the audible alarm triggered by the alarm condition upon receiving the tactile input on the alarm icon 820 or the alarm ribbon 822. This is shown in FIG. 8 where the alarm ribbon 822 has a countdown timer 850 that shows two minutes remaining for pausing the audible alarm caused by the detected alarm condition. When the countdown timer 850 reaches zero, the speaker 132 resumes generating the audible alarm on the medical device 100.

The alarm pause menu 830 includes a plurality of available audible alarm pause options. The alarm pause menu 830 includes a first alarm pause 832 having a first duration of three minutes, a second alarm pause 834 having a second duration of five minutes, a third alarm pause 836 having a third duration of seven minutes, and a fourth alarm pause 838 having a fourth duration of ten minutes. Each of the first, second, third, and fourth durations shown in the alarm pause menu 830 are longer than the default duration of two minutes for the default alarm pause 824. The first, second, third, and fourth durations are preset for selection on the display 104. The first, second, third, and fourth durations are preset not to exceed a maximum duration.

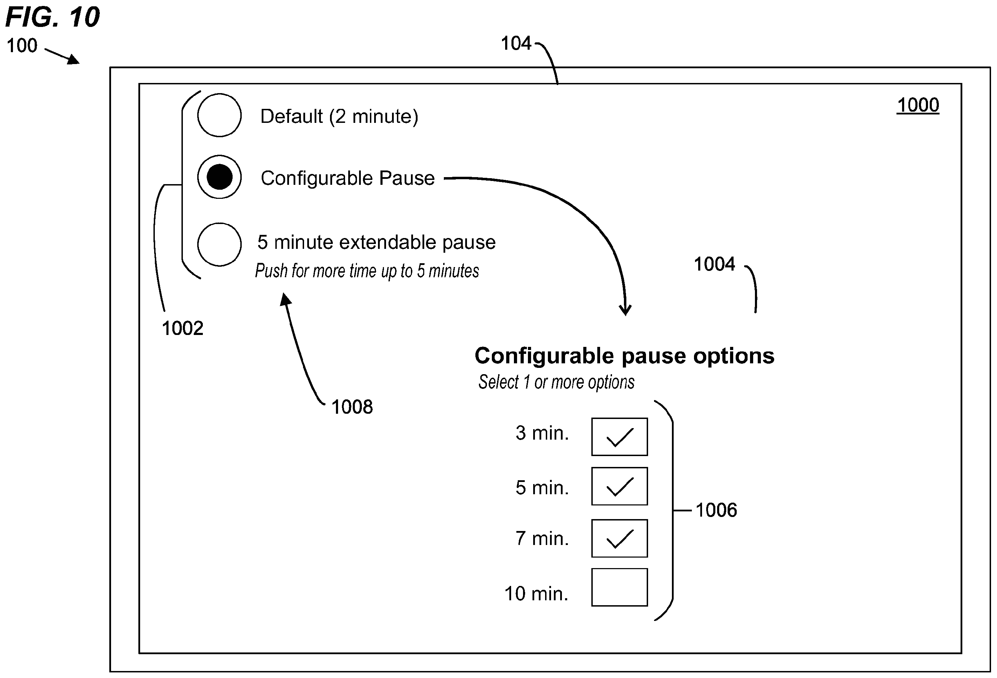

The first, second, third, and fourth durations are provided for illustrative purposes and it is contemplated that the alarm pause tool 126 may provide fewer audible alarm pause options, or more audible alarm pause options. Further, the durations of the audible alarm pause options may vary. As will be described in more detail further below, the quantity of audible alarm pause time options, as well as the duration for each audible alarm pause time option, may be configured by using a configuration user interface 1000 shown in FIG. 10.

Referring back to FIG. 6, in response to receiving a selection in the alarm pause menu 830, the method 600 includes an operation 608 of controlling the speaker 132 to pause the audible alarm for an extended duration. The extended duration is based on whether the first alarm pause 832, the second alarm pause 834, the third alarm pause 836, or the fourth alarm pause 838 is selected in the alarm pause menu 830. As discussed above, each of the first, second, third, and fourth durations of the first alarm pause 832, the second alarm pause 834, the third alarm pause 836, or the fourth alarm pause 838, respectively, is longer than the default duration (e.g., two minutes) of the default alarm pause 824.

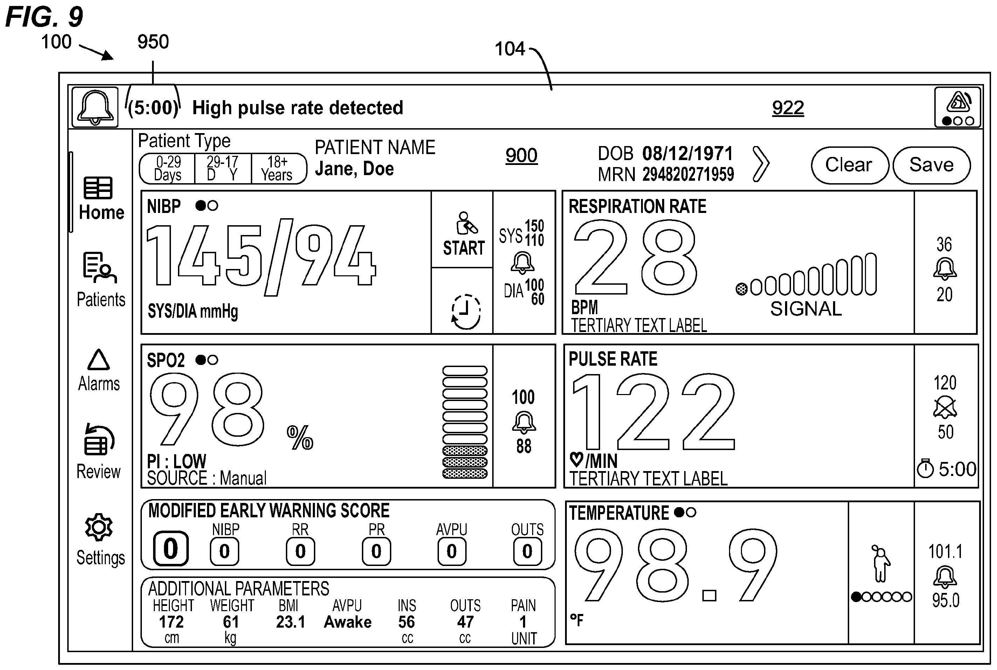

FIG. 9 illustrates another example of a home screen user interface 900 that can be displayed on the display 104 of the medical device 100 as a result of a selection of the second alarm pause 834 having the second duration of five minutes in the alarm pause menu 830 of FIG. 8. Upon selection of one of the first, second, third, and fourth alarm pauses 832-838 in the alarm pause menu 830, an extended duration (i.e., one of the first, second, third, and fourth durations) is applied for silencing the audible alarm triggered by the alarm condition.

This is shown in FIG. 9 where the countdown timer 950 in the alarm ribbon 922 is updated to show that there are five minutes remaining for pausing the audible alarm caused by the detected alarm condition. As the countdown timer 950 ticks down to zero, the user of medical device 100 can extend the pause of the audible alarm further by selecting one of the first, second, third, and fourth alarm pauses 832-838 in the alarm pause menu 830. Also, the user can select a resume option 840 that when selected on the alarm pause menu 830 causes the medical device 100 to resume the default alarm pause 824 having the default duration. Accordingly, the default and/or extended audible alarm pauses can be extended at any time by a user of the medical device 100 including before expiration of the default and/or extended audible alarm pauses.

In some examples, operation 608 of controlling the speaker 132 to pause the audible alarm for an extended duration is based on an audible input instead of a tactile input. For example, the configurable audible alarm pause can be paired with voice recognition to remove the need to physically interact with the display 104 of the medical device 100 to select one of the extended durations for audible alarm pause in the alarm pause menu 830. Advantageously, this can expedite response and interaction with the patient by a user of the medical device 100.

In some examples, an external device such as a wearable badge has a speaker and microphone unit that detects speech from a wearer of the badge, and that is equipped with a voice recognition to understand and interpret the speech. The wearable badge communicates instructions to the medical device 100 such as wireless signals received by the user presence sensor 136 to cause the speaker 132 on the medical device 100 to pause the audible alarm for a custom duration based on the audible input received from the wearer of the wearable badge.

Alternatively, the medical device 100 can be equipped with a microphone to detect speech from an authorized user, and can be further equipped with voice recognition to understand and interpret the speech such that the medical device 100 can pause the audible alarm for a custom duration based on an audible input without using an external device.

The custom duration for the audible alarm pause based on the audible input can be longer than or equal to the default duration of the default alarm pause 824, and is shorter than a maximum duration to protect patient safety. In some examples, the custom duration lasts as long as the authorized user is within a threshold distance of the medical device 100. For example, the wearable badge worn by the authorized user can emit a wireless signal that is detected by the user presence sensor 136 of the medical device 100 when the authorized user is within the threshold distance of the medical device 100. When the authorized user moves outside of the threshold distance, the wireless signal is no longer detectable by the user presence sensor 136 such that the medical device 100 can determine that the authorized user is no longer within the threshold distance. When the medical device 100 detects the authorized user is no longer within the threshold distance, the medical device 100 can resume the default alarm pause 824 and/or generate the audible alarm when the alarm condition persists.

Referring back to FIG. 6, the method 600 can include an operation 610 of resuming the default alarm pause 824. In some examples, the default alarm pause 824 is resumed based on an input received on the medical device 100. As shown in the example of FIG. 8, the alarm pause menu 830 includes the resume option 840 that when selected on the alarm pause menu 830 causes the medical device 100 to resume the default alarm pause 824 having the default duration. Accordingly, the extended audible alarm pause having one of the first, second, third, or fourth durations can be canceled at any time by a user of the medical device 100 including before expiration of the extended audible alarm pause to revert back to the default alarm pause 824.

FIG. 10 illustrates an example of a configuration user interface 1000 that can be displayed on the display 104 of the medical device 100 to configure the alarm pause tool 126. In this example, the configuration user interface 1000 includes a first set of inputs 1002 that are selectable to configure the default alarm pause, the configurable alarm pause, and a five minute extendible alarm pause. The first set of inputs 1002 include graphical user interface elements (e.g., small circles) that allow users to select one option from a group of mutually exclusive options. When a graphical user interface element is selected, a dot or marker appears inside the element to indicate the selection. Clicking on an element selects it and deselects the other elements within the first set of inputs 1002. In FIG. 10, the configurable alarm pause is shown as being selected in the first set of inputs 1002 of the configuration user interface 1000.

As further shown in FIG. 10, the configuration user interface 1000 displays a configurable pause options window 1004 for the configurable alarm pause selected in the first set of inputs 1002. The configurable pause options window 1004 allows an authorized user such as an administrator to select one or more extended alarm pause options 1006 for display in the alarm pause menu 830. For example, the configurable pause options window 1004 includes four alarm pause options: a first alarm pause having a first duration of three minutes, a second alarm pause having a second duration of five minutes, a third alarm pause having a third duration of seven minutes, and a fourth alarm pause having a fourth duration of ten minutes. In FIG. 10, the first, second, and third alarm pauses are shown as selected, while the fourth alarm pause is not selected. This will cause the alarm pause menu 830 to display the first, second, and third alarm pauses for selection by a user of the medical device 100, but not the fourth alarm pause.

In view of the example shown in FIG. 10, the durations of the first, second, third, and fourth alarm pauses are preset such as by a manufacturer of the medical device 100. It is contemplated that in alternative examples, the durations of the first, second, third, and fourth alarm pauses can be configurable by an authorized user such as an administrator. For example, in such alternative examples, an administrator can configure the first alarm pause to have a duration of four minutes instead of the three minutes shown in the example of FIG. 10.

Further, the first, second, third, and fourth durations are preset not to exceed a maximum duration. For example, the fourth alarm pause having a duration of ten minutes can be considered as a maximum duration for pausing the audible alarms on the medical device 100 because any pause longer than ten minutes could be considered unsafe for monitoring patients. The maximum duration can be preset by a manufacturer of the medical device 100.

As further shown in FIG. 10, the configuration user interface 1000 includes a configurable audible alarm pause option 1008 that can be set up by a facility administrator to allow a user of the medical device 100 to press for "more time" on the display 104, which causes the speaker 132 to pause the audible alarm up to a preset time limit (e.g., five minutes).

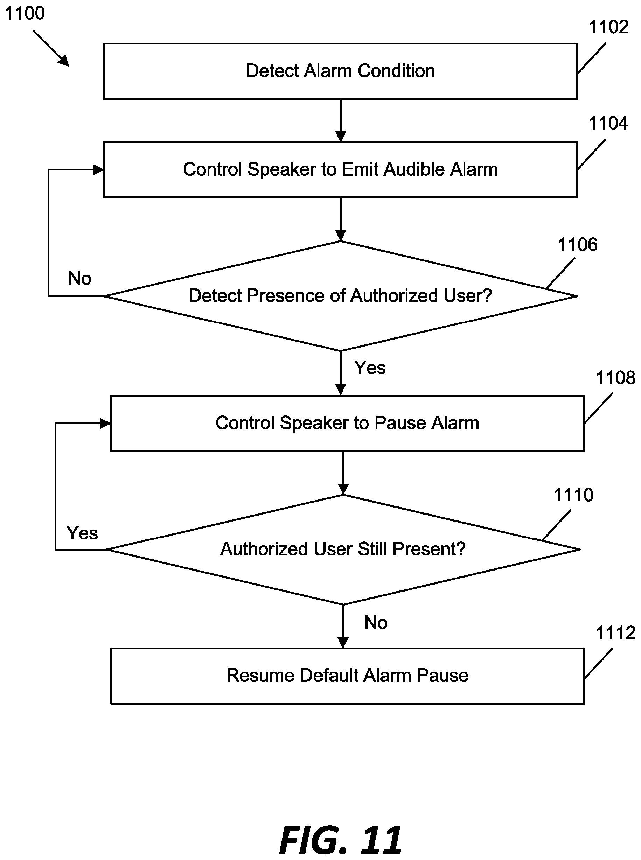

FIG. 11 schematically illustrates another example of a method 1100 of managing audible alarms on the medical device 100. The method 1100 can be performed by the alarm pause tool 126 when executed on the medical device 100. Like the method 600 of FIG. 6, the method 1100 can be executed on the medical device 100 to pause audible alarms triggered by measurements of one or more of SpO2, pulse rate, respiration rate, and NIBP.

The method 1100 includes an operation 1102 of detecting an alarm condition based on data from the one or more sensors communicatively coupled to the medical device 100 such as the NIBP sensor 106, the thermometer module 108, and the pulse oximeter sensor 112. Operation 1102 can include determining measurement values of the one or more physiological variables based on the data received from the sensors communicatively coupled to the medical device 100, and comparing the measurement values to upper and lower alarm limits. For example, operation 1102 can include detecting an alarm condition when the measurement values exceed the upper alarm limit or when the measurement values are less than the lower alarm limit.

The method 1100 includes an operation 1104 of controlling the speaker 132 to emit an audible alarm based on the alarm condition detected in operation 1102. For example, when the measurements of a physiological variable are higher than an upper alarm limit or when the measurements of a physiological variable are lower than a lower alarm limit, the speaker 132 is controlled to emit an audible alarm to alert a user of the medical device 100 such as a clinician, nurse, physician, or other type of caregiver about the alarm condition.

The method 1100 includes an operation 1106 of determining whether an authorized user is within a threshold distance of the medical device 100. The presence of the authorized user can be based on detection of a signal by the user presence sensor 136. For example, the authorized user can wear a badge that emits a wireless signal that is detectable by the user presence sensor 136 so long as the authorized user is within the threshold distance of the medical device 100. Illustrative examples of the wireless signals that can be emitted by the badge worn by the authorized user can include, without limitation, Wi-Fi, Bluetooth, Zigbee, radio frequency identification (RFID), Z-Wave, LoRaWAN, or other wireless signals.

When the presence of an authorized user is not detected in operation 1106 (i.e., "No" in operation 1106), the method 1100 returns to operation 1104 such that the speaker 132 continues to emit the audible alarm. When the presence of an authorized user is detected in operation 1106 (i.e., "Yes" in operation 1106), the method 1100 proceeds to an operation 1108 of controlling the speaker 132 to pause the audible alarm for an extended audible alarm pause. In some examples, the extended audible alarm pause is preset for five minutes. After the extended audible alarm pause expires, the authorized user can extend the alarm pause such as by selecting one of the options in the alarm pause menu 830 via tactile input on the display 104, or by uttering a spoken command to extend the alarm pause via audible input.

The method 1100 includes an operation 1110 of determining whether the authorized user is still within the threshold distance of the medical device 100. Operation 1110 can be based on whether the user presence sensor 136 continues to receive the wireless signal from the badge worn by the authorized user. As discussed above, when the authorized user walks away from the medical device 100 such that the authorized user is no longer within the threshold distance of the medical device 100, the user presence sensor 136 is no longer able to receive the wireless signal from the badge worn by the authorized user such that it can be determined in operation 1110 that the authorized user is no longer within the threshold distance of the medical device 100.

When the presence of the authorized user is detected to still be within the threshold distance of the medical device 100 (i.e., "Yes" in operation 1110), the method 1100 returns to operation 1108 such that the speaker 132 continues to pause the audible alarm for the extended audible alarm pause. When the authorized user moves outside of the threshold distance of the medical device 100 (i.e., "No" in operation 1110), the method 1100 proceeds to an operation 1112 of resuming a default alarm pause that when expired causes the speaker 132 to generate the audible alarm when the alarm condition continues to be detected by the medical device 100.

In view of the foregoing, the alarm pause tool 126 enables configuration of available alarm pause options. Manufacturer limitations may be placed on a total length of time that an audible alarm may be paused or silenced on the medical device 100 even when the available alarm pause options are configurable. The pause of audible alarms by the alarm pause tool 126 may be set as parameter-specific (i.e., current alarm only) or for the whole-device such that audible alarm pause options may be configured to function as a single audible alarm pause or a whole device (i.e., multiple alarm) audible alarm pause. The alarm pause tool 126 can be used with or without the pairing of user location such that an automatic audible alarm restart can occur upon an authorized user (e.g., clinician) moving out of range of the medical device 100. Further, the alarm pause tool 126 can be paired with voice recognition to remove physical interaction with the display 104 of the medical device 100 to expedite response and interaction with the patient. Also, the alarm pause tool 126 enables configurable audible alarm pause on the medical device 100 that can be set up by a facility administrator to allow for a press for "more time" on the audible alarm pause up to a specific time limit (e.g., five minutes).

The alarm pause tool 126 advantageously allows different areas or units within a healthcare facility such as a hospital to choose appropriate pauses for audible alarm as needed and as defined by the healthcare facility. Advantageously, this can limit disruption of healthcare caused by audible alarms that are redundant such as audible alarms that have already been acknowledged and are being addressed by an authorized user such as a clinician.

The audible alarm pause options are displayed at the point of alarm instance (i.e., on the medical device 100). This allows the audible alarm pause to be dynamic based on a particular context or situation. For example, a user of the medical device 100 such as a clinician can choose an appropriate amount of time for pausing an audible alarm based on the patient's condition and the intervention needed at a particular instant, providing more flexibility for the clinician.

Thus, the alarm pause tool 126 reduces the likelihood of redundant audible alarms disrupting bedside care, allows flexibility to set appropriate audible alarm pause options that match the acuity of care needed for a particular context or situation, and allows for optional pairing to user location for resuming an audible alarm upon a user leaving the patient unattended when the alarm condition persists, and/or returning to a default audible alarm pause.

The alarm pause tool 126 provides specific technical improvements to medical device alarm management systems by implementing a configurable multi-duration alarm pause capability that reduces unnecessary disruptions while maintaining patient safety. The tool addresses the concrete technical problem of alarm fatigue in healthcare environments by providing an intelligent and customizable approach to managing audible medical alarms.

Rather than using conventional fixed-duration alarm pauses, the alarm pause tool 126 implements a technical solution on the medical device 100 that dynamically adapts alarm management based on multiple factors including user presence detection through wireless signals, customizable duration options, and automated resumption of default audible alarm pause. These features, whether viewed independently of one another or in combination, provide specific improvements in the technical field of medical device interfaces and alarm management.

The alarm pause tool 126 provides a practical application by integrating multiple technical elements including wireless presence detection, touchscreen interfaces, and speaker control in a specific configuration that improves both the functioning of the medical device 100 and the ability of healthcare providers to deliver healthcare. The technical implementation reduces cognitive burden on healthcare workers while maintaining critical safety monitoring.

The combination of wireless presence detection, configurable audible alarm pause durations, and automated resumption of default settings provides significantly more than the routine or conventional use of general purpose computers or medical devices. The system implements a particular solution to the technological problem of managing medical device alarms in a way that improves both technical operation and practical utility.

The alarm pause tool 126 when executed on the medical device 100 enables specific improvements including: reduced disruptions during patient care through intelligent pause duration options, enhanced safety through automated resumption based on user presence detection, and improved alarm management workflow through a configurable interface. These represent concrete technical benefits rather than mere automation of manual processes.

Embodiments of the present disclosure can be described with reference to the following numbered clauses, with preferred features laid out in the dependent clauses:
1. A medical device for monitoring one or more physiological variables, the medical device comprising:
   a display configured to display measurements of the one or more physiological variables;
   a speaker configured to emit an audible alarm when the measurements of the one or more physiological variables are higher than an upper alarm limit or when the measurements of the one or more physiological variables are lower than a lower alarm limit;
   at least one processing device communicatively coupled to the display, the speaker, and at least one sensor that captures the measurements of the one or more physiological variables; and
   a memory device storing instructions which, when executed by the at least one processing device, cause the at least one processing device to:
      control the speaker to emit the audible alarm when the measurements of the one or more physiological variables are higher than the upper alarm limit or when the measurements of the one or more physiological variables are lower than the lower alarm limit;
      in response to receiving a first input, display a menu that includes at least a first alarm pause having a first duration and a second alarm pause having a second duration that is different from the first duration; and
      in response to receiving a second input, control the speaker to pause the audible alarm for the first duration or the second duration.
2. The medical device of clause 1, wherein the first and second durations differ from a default duration for a default alarm pause.
3. The medical device of clause 2, wherein the memory device stores additional instructions which, when executed by the at least one processing device, further cause the at least one processing device to:
   resume the default alarm pause in response to receiving a third input.
4. The medical device of any one of clauses 1-3, wherein the menu includes a third alarm pause having a third duration that is different from the first and second durations.
5. The medical device of clause 4, wherein the menu includes a fourth alarm pause having a fourth duration that is different from the first, second, and third durations.
6. The medical device of clause 5, wherein the first, second, third, and fourth durations are preset for selection not to exceed a maximum duration.
7. The medical device of any preceding clause, further comprising:
   a user presence sensor; and
   wherein the memory device stores additional instructions which, when executed by the at least one processing device, further cause the at least one processing device to:
      detect a presence of an authorized user within a threshold distance of the medical device based on detection of a signal by the user presence sensor; and
      control the speaker to pause the audible alarm while the authorized user remains within the threshold distance of the medical device for an extended duration.
8. The medical device of clause 7, wherein the memory device stores additional instructions which, when executed by the at least one processing device, further cause the at least one processing device to:
   resume a default alarm pause when the authorized user moves outside of the threshold distance of the medical device.
9. The medical device of any preceding clause, wherein the memory device stores additional instructions which, when executed by the at least one processing device, further cause the at least one processing device to:
   control the speaker to pause the audible alarm for a custom duration based on an audible input, wherein the custom duration is longer than or equal to a default duration for a default alarm pause and is shorter than a maximum duration.
10. The medical device of any one of clauses 1-9, wherein the pause of the audible alarm is applied to one or more of blood oxygen saturation (SpO2), pulse rate, respiration rate, and non-invasive blood pressure.
11. A medical device for monitoring one or more physiological variables, the medical device comprising:
   a user presence sensor;
   a speaker configured to emit an audible alarm when measurements of the one or more physiological variables are higher than an upper alarm limit or when the measurements of the one or more physiological variables are lower than a lower alarm limit;
   at least one processing device communicatively coupled to the user presence sensor, the speaker, and at least one sensor that captures the measurements of the one or more physiological variables; and
   a memory device storing instructions which, when executed by the at least one processing device, cause the at least one processing device to:
      detect a presence of an authorized user within a threshold distance of the medical device based on detection of a signal by the user presence sensor;
      control the speaker to pause the audible alarm for an extended duration when the authorized user remains within the threshold distance of the medical device; and
      resume a default alarm pause when the authorized user moves outside of the threshold distance of the medical device.
12. The medical device of clause 11, wherein the memory device stores additional instructions which, when executed by the at least one processing device, further cause the at least one processing device to:
   in response to receiving a first input, display a menu that includes at least a first alarm pause having a first duration and a second alarm pause having a second duration that is different from the first duration; and
   in response to receiving a second input, control the speaker to pause the audible alarm for the first duration or the second duration, wherein the first and second durations differ from a default duration for the default alarm pause.
13. The medical device of clause 12, wherein the memory device stores additional instructions which, when executed by the at least one processing device, further cause the at least one processing device to:
   resume the default alarm pause in response to receiving a third input.
14. The medical device of clause 12 or 13, wherein the menu includes a third alarm pause having a third duration that is different from the first and second durations; and
   wherein the menu includes a fourth alarm pause having a fourth duration that is different from the first, second, and third durations.
15. The medical device of any one of clauses 11-14, wherein the pause of the audible alarm is applied to at least one of blood oxygen saturation (SpO2), pulse rate, respiration rate, and non-invasive blood pressure.
16. A medical device for monitoring one or more physiological variables, comprising:
   a speaker configured to emit an audible alarm when measurements of the one or more physiological variables are higher than an upper alarm limit or when the measurements of the one or more physiological variables are lower than a lower alarm limit;
   at least one processing device communicatively coupled to the speaker and to at least one sensor that captures the measurements of the one or more physiological variables; and
   a memory device storing instructions which, when executed by the at least one processing device, cause the at least one processing device to:
      control the speaker to pause the audible alarm for a custom duration based on an audible input, wherein the custom duration is longer than or equal to a default duration for a default alarm pause and is shorter than a maximum allowed duration, and wherein the custom duration lasts as long as an authorized user is within a threshold distance of the medical device.
17. The medical device of clause 16, wherein the memory device stores additional instructions which, when executed by the at least one processing device, further cause the at least one processing device to:
   in response to receiving a first input, display a menu that includes at least a first alarm pause having a first duration and a second alarm pause having a second duration that is different from the first duration; and
   in response to receiving a second input, control the speaker to pause the audible alarm for the first duration or the second duration, wherein the first and second durations differ from the default duration for the default alarm pause.
18. The medical device of clause 17, wherein the memory device stores additional instructions which, when executed by the at least one processing device, further cause the at least one processing device to:
   in response to receiving a third input, control the speaker to resume the default alarm pause.
19. The medical device of clause 17 or 18, wherein the menu includes a third alarm pause having a third duration that is different from the first and second durations; and
   wherein the menu includes a fourth alarm pause having a fourth duration that is different from the first, second, and third durations.
20. The medical device of any one of clauses 16-19, wherein the pause of the audible alarm is applied to one or more of blood oxygen saturation (SpO2), pulse rate, and respiration rate.
21. A method of monitoring one or more physiological variables, the method comprising:
   controlling a speaker to emit an audible alarm when measurements of the one or more physiological variables are higher than an upper alarm limit or when the measurements of the one or more physiological variables are lower than a lower alarm limit;
   in response to receiving a first input, displaying a menu that includes at least a first alarm pause having a first duration and a second alarm pause having a second duration that is different from the first duration; and
   in response to receiving a second input, controlling the speaker to pause the audible alarm for the first duration or the second duration.
22. The method of clause 21, wherein the first and second durations differ from a default duration for a default alarm pause.
23. The method of clause 22, further comprising:
   resuming the default alarm pause in response to receiving a third input.
24. The method of any one of clauses 21-23, wherein the menu includes a third alarm pause having a third duration that is different from the first and second durations.
25. The method of clause 24, wherein the menu includes a fourth alarm pause having a fourth duration that is different from the first, second, and third durations.
26. The method of clause 25, wherein the first, second, third, and fourth durations are preset for selection on a touchscreen display.
27. The method of clause 21, further comprising:
   detecting a presence of an authorized user within a threshold distance based on detection of a signal by a user presence sensor; and
   controlling the speaker to pause the audible alarm while the authorized user remains within the threshold distance of the medical device.
28. The method of clause 27, further comprising:
   resuming a default alarm pause when the authorized user moves outside of the threshold distance of the medical device.
29. The method of clause 21, further comprising:
   controlling the speaker to pause the audible alarm for a custom duration based on an audible input, wherein the custom duration is longer than or equal to a default duration for a default alarm pause and is shorter than a maximum duration.
30. The method of any one of clauses 21-29, wherein the pause of the audible alarm is applied to one or more of blood oxygen saturation (SpO2), pulse rate, respiration rate, and non-invasive blood pressure.

## Claims

1. A medical device for monitoring one or more physiological variables, the medical device comprising:
a display configured to display measurements of the one or more physiological variables;
a speaker configured to emit an audible alarm when the measurements of the one or more physiological variables are higher than an upper alarm limit or when the measurements of the one or more physiological variables are lower than a lower alarm limit;
at least one processing device communicatively coupled to the display, the speaker, and at least one sensor that captures the measurements of the one or more physiological variables; and
a memory device storing instructions which, when executed by the at least one processing device, cause the at least one processing device to:
control the speaker to emit the audible alarm when the measurements of the one or more physiological variables are higher than the upper alarm limit or when the measurements of the one or more physiological variables are lower than the lower alarm limit;
in response to receiving a first input, display a menu that includes at least a first alarm pause having a first duration and a second alarm pause having a second duration that is different from the first duration; and
in response to receiving a second input, control the speaker to pause the audible alarm for the first duration or the second duration.

2. The medical device of claim 1, wherein the first and second durations differ from a default duration for a default alarm pause.

3. The medical device of claim 2, wherein the memory device stores additional instructions which, when executed by the at least one processing device, further cause the at least one processing device to:
resume the default alarm pause in response to receiving a third input.

4. The medical device of any one of claims 1-3, wherein the menu includes a third alarm pause having a third duration that is different from the first and second durations, and a fourth alarm pause having a fourth duration that is different from the first, second, and third durations.

5. The medical device of claim 4, wherein the first, second, third, and fourth durations are preset for selection not to exceed a maximum duration.

6. The medical device of any preceding claim, further comprising:
a user presence sensor; and
wherein the memory device stores additional instructions which, when executed by the at least one processing device, further cause the at least one processing device to:
detect a presence of an authorized user within a threshold distance of the medical device based on detection of a signal by the user presence sensor; and
control the speaker to pause the audible alarm for an extended duration while the authorized user remains within the threshold distance of the medical device.

7. The medical device of claim 6, wherein the memory device stores additional instructions which, when executed by the at least one processing device, further cause the at least one processing device to:
resume a default alarm pause when the authorized user moves outside of the threshold distance of the medical device.

8. The medical device of any preceding claim, further comprising:
a user presence sensor; and
wherein the memory device stores additional instructions which, when executed by the at least one processing device, further cause the at least one processing device to:
control the speaker to pause the audible alarm for a custom duration based on an audible input, wherein the custom duration is longer than or equal to a default duration for a default alarm pause and is shorter than a maximum allowed duration, and wherein the custom duration lasts as long as an authorized user is detected by the user presence sensor as being within a threshold distance of the medical device.

9. The medical device of any one of claims 1-8, wherein the pausing of the audible alarm is applied to one or more of blood oxygen saturation (SpO2), pulse rate, respiration rate, and non-invasive blood pressure.

10. A method of monitoring one or more physiological variables, the method comprising:
controlling a speaker to emit an audible alarm when measurements of the one or more physiological variables are higher than an upper alarm limit or when the measurements of the one or more physiological variables are lower than a lower alarm limit;
in response to receiving a first input, displaying a menu that includes at least a first alarm pause having a first duration and a second alarm pause having a second duration that is different from the first duration; and
in response to receiving a second input, controlling the speaker to pause the audible alarm for the first duration or the second duration.

11. The method of claim 10, wherein the first and second durations differ from a default duration for a default alarm pause.

12. The method of claim 11, further comprising:
resuming the default alarm pause in response to receiving a third input.

13. The method of any one of claims 10-12, wherein the menu includes a third alarm pause having a third duration different from the first and second durations, and a fourth alarm pause having a fourth duration different from the first, second, and third durations, wherein the first, second, third, and fourth durations are preset for selection on a touchscreen display.

14. The method of any one of claims 10 to 13, further comprising:
detecting a presence of an authorized user within a threshold distance based on detection of a signal by a user presence sensor;
controlling the speaker to pause the audible alarm while the authorized user remains within the threshold distance; and
resuming a default alarm pause when the authorized user moves outside of the threshold distance.

15. The method of any one of claims 10 to 14, further comprising:
controlling the speaker to pause the audible alarm for a custom duration based on an audible input, wherein the custom duration is longer than or equal to a default duration for a default alarm pause and is shorter than a maximum duration.
